# EUROPEAN PATENT APPLICATION

(11) **EP 4 062 899 A1**
(43) Date of publication of application: **28.09.2022**
(21) Application number: 20890639.6
(22) Date of filing: 18.11.2020
(51) Int. Cl.: A61K 8/894, A61K 8/06, A61K 8/31, A61K 8/37, A61K 8/92, A61Q 1/02, A61Q 17/04, A61Q 19/00

(54) **WATER-IN-OIL COSMETIC PREPARATION**

(30) Priority: 19.11.2019 JP 2019208392
(71) Applicant: Shin-Etsu Chemical Co., Ltd., Tokyo 100-0005 (JP)
(72) Inventor: MIYAUCHI Masaru, Tokyo 100-0004 (JP); KONISHI Masayuki, Tokyo 100-0004 (JP)
(74) Representative: Mewburn Ellis LLP
(86) International application number: PCT/JP2020/042972
(87) International publication number: WO 2021/100750

(57) **Abstract**

The present invention provides a water-in-oil cosmetic preparation which contains:
(a) from 0.1% by mass to 3% by mass of a substance that is selected from among alkyl-modified/partially crosslinked polyether-modified silicones and alkyl-modified/partially crosslinked polyglycerol-modified silicones;
(b) from 0.05% by mass to 0.8% by mass of an uncrosslinked alkyl branched polyether-modified silicone;
(c) from 30% by mass to 90% by mass of an aqueous component; and
(d) from 5% by mass to 30% by mass of an oil component.

This water-in-oil cosmetic preparation has excellent emulsion stability even in cases where an oil component such as a hydrocarbon oil or an ester oil is contained therein in a large amount, while exhibiting good spreadability since the emulsified state thereof is smoothly broken upon application, and enabling the achievement of water breaking sensation and excellent feeling of use.

## Description

### TECHNICAL FIELD

This invention relates to a water-in-oil cosmetic composition having at least one oil selected from hydrocarbon oils, ester oils and plant oils, blended in the oil phase. Herein, the cosmetic composition is also referred to as cosmetics.

### BACKGROUND ART

With the aim to formulate a W/O cosmetic composition having a fresh feeling on use, it is known from Patent Document 1 that an emulsion having a high water content and a large particle size is prepared using a partially crosslinked polyether-modified silicone. From this emulsion, a cosmetic composition having a fresh feeling on use is obtained. It is also known from Patent Document 2 that a cosmetic composition of water break type is prepared by designing an emulsion having a large particle size.

Among cosmetic compositions of water break type, those compositions having silicone oil, hydrocarbon oil and ester oil blended in the oil phase are known from Patent Document 3. The cosmetic compositions of water break type having large amounts of hydrocarbon oil and ester oil blended are difficult to stabilize. When large amounts of surfactants are blended in order to enhance stability, there arise problems on use such as shortage of freshness, heavy spreading and sticky texture (see Patent Documents 4 and 5).

### PRIOR ART DOCUMENTS

### PATENT DOCUMENTS

Patent Document 1: JP-A 2001-2521
Patent Document 2: JP-A 2004-502715
Patent Document 3: JP 3782914
Patent Document 4: JP 5156322
Patent Document 5: JP 4906520

### SUMMARY OF INVENTION

### TECHNICAL PROBLEM

An object of the invention, which has been made under the above-mentioned circumstances, is to provide a W/O cosmetic composition which even when a large amount of an oil component such as hydrocarbon oil or ester oil is blended, is improved in emulsion stability, allows the emulsion state to be smoothly broken upon application, is well spreadable, and gives a water-breaking sensation with a pleasant feeling on use. As used herein, the term "water break" refers to the phenomenon that shearing forces acting on a cosmetic preparation upon application decompose or break the W/O emulsion whereby the water phase or internal phase bursts out as watery droplets.

### SOLUTION TO PROBLEM

Making extensive investigations to attain the above object, the inventors have found that the outstanding problems are solved by formulating a W/O cosmetic composition comprising (a) at least one silicone selected from among alkyl-modified, partially crosslinked polyether-modified silicones and alkyl-modified, partially crosslinked polyglycerin-modified silicones, (b) a non-crosslinked alkyl-branched polyether-modified silicone, (c) an aqueous component, and (d) an oil component. The invention is predicated on this finding.

Accordingly, the invention provides a water-in-oil cosmetic composition as defined below.
[1] A water-in-oil cosmetic composition comprising:
   (a) 0.1 to 3% by weight of at least one silicone selected from among alkyl-modified, partially crosslinked polyether-modified silicones and alkyl-modified, partially crosslinked polyglycerin-modified silicones,
   (b) 0.05 to 0.8% by weight of a non-crosslinked alkyl-branched polyether-modified silicone,
   (c) 30 to 90% by weight of an aqueous component, and
   (d) 5 to 30% by weight of an oil component exclusive of silicone oil.
[2] The water-in-oil cosmetic composition of [1], further comprising (e) an alkyl-modified, partially crosslinked organopolysiloxane exclusive of component (a).
[3] The water-in-oil cosmetic composition of [1] or [2] wherein component (d) contains a hydrocarbon oil and a low polar ester oil in a total amount of at least 80% by weight.
[4] The water-in-oil cosmetic composition of any one of [1] to [3] wherein component (a) is a swollen product which is previously swollen in a hydrocarbon oil.
[5] The water-in-oil cosmetic composition of any one of [1] to [4] wherein component (b) is cetyl PEG/PPG-10/1 dimethicone as defined by the INCI name.

### ADVANTAGEOUS EFFECTS OF INVENTION

The invention provides the W/O cosmetic composition which even when a large amount of an oil component such as hydrocarbon oil or ester oil is blended, is improved in emulsion stability, allows the emulsified state to be smoothly broken upon application, is well spreadable, and gives a water-breaking sensation with a pleasant feeling on use.

### DESCRIPTION OF EMBODIMENTS

Now the invention is described in detail. It is noted that the water-in-oil cosmetic composition is simply referred to as cosmetic or cosmetic composition, hereinafter. Some compounds are described by the Japanese labeling name or the International Nomenclature for Cosmetic Ingredients (INCI).

### [Component (a)]

Component (a) is at least one silicone selected from among alkyl-modified, partially crosslinked polyether-modified silicones and alkyl-modified, partially crosslinked polyglycerin-modified silicones. The alkyl-modified, partially crosslinked polyether-modified silicones are three-dimensional crosslinked products obtained by crosslinking an organopolysiloxane chain having alkyl-modifying chains on a silicone backbone with polyether groups. Examples of the alkyl-modified, partially crosslinked polyether-modified silicone include known ones defined by labeling names, such as PEG-15/lauryl dimethicone crosspolymer (labeling name, INCI name: PEG-15/Lauryl Dimethicone Crosspolymer), and PEG-15/lauryl polydimethylsiloxyethyl dimethicone crosspolymer (labeling name, INCI name: PEG-15/Lauryl Polydimethylsiloxyethyl Dimethicone Crosspolymer). Those of the type wherein branches from the backbone are solely alkyl are preferred from the aspect of compatibility (compatibility between components, simply referred to as compatibility, hereinafter). Specifically, they are commercially available as swollen products containing mineral oil (labeling name, INCI name: Mineral Oil) or other oils, for example, under the tradename of KSG-310, 320, 330, and 340 (all from Shin-Etsu Chemical Co., Ltd.).

The alkyl-modified, partially crosslinked polyglycerin-modified silicones are three-dimensional crosslinked products obtained by crosslinking an organopolysiloxane chain having alkyl-modifying chains on a silicone backbone with polyglycerin groups. Examples of the alkyl-modified, partially crosslinked polyglycerin-modified silicone include known ones defined by labeling names, such as lauryl dimethicone/polyglycerin-3 crosspolymer (labeling name, INCI name: Lauryl Dimethicone/Polyglycerin-3 Crosspolymer), and polyglyceryl-3/lauryl polydimethylsiloxyethyl dimethicone crosspolymer (labeling name, INCI name: Polyglyceril-3/Lauryl Polydimethylsiloxyethyl Dimethicone Crosspolymer). Those of the type wherein branches from the backbone are solely alkyl are preferred from the aspect of compatibility. Specifically, they are commercially available as swollen products, typically gels, containing hydrocarbon oils, mineral oil (labeling name, INCI name: Mineral Oil) or other oils, for example, under the tradename of KSG-810, 820, 830, and 840 (all from Shin-Etsu Chemical Co., Ltd.).

As component (a), the alkyl-modified, partially crosslinked polyether-modified silicones are preferred in view of emulsion stability improvement. Swollen products of these silicones swollen in nonpolar oils such as hydrocarbon oils are more preferred in view of emulsion stability and compatibility. They are commercially available, for example, under the tradename of KSG-310, 320, and 340 (all from Shin-Etsu Chemical Co., Ltd.). It is noted that examples of the hydrocarbon oil include those described later for component (d).

The amount of component (a) blended is 0.1 to 3% by weight, preferably 0.2 to 1.2% by weight of the overall cosmetic composition. If the amount is less than 0.1% by weight, the cosmetic composition has low emulsion stability. If the amount exceeds 3% by weight, the cosmetic composition loses watery refreshing sensation, detracting from feeling on use. Notably, when component (a) is a swollen product, the amount is the amount of solids (or net weight) of component (a).

Component (a) may be used alone or in combination of two or more. On use of a mixture of an alkyl-modified, partially crosslinked polyether-modified silicone with an alkyl-modified, partially crosslinked polyglycerin-modified silicone, for example, when the relative blend ratio of the alkyl-modified, partially crosslinked polyether-modified silicone is high, the cosmetic composition is more prone to spread. When the relative blend ratio of the alkyl-modified, partially crosslinked polyglycerin-modified silicone is high, the cosmetic composition tends to provide a moist soft feeling on use. In controlling the feeling on use, the blend ratio may be determined as appropriate.

### [Component (b)]

Component (b) used herein is a non-crosslinked alkyl-branched polyether-modified silicone. Specifically, it is a non-crosslinked silicone based on an organopolysiloxane (e.g., dimethicone) backbone and structured to have a hydrophilic group in the form of a polyalkylene ether group and a hydrophobic portion in which some constituent -CH₃ directly bonded to a silicon atom in the organopolysiloxane is substituted by a hydrocarbon chain of the formula -CₙH(₂ₙ₊₁) wherein n>2, the organopolysiloxane backbone being free of silicone branches. Examples include linear polyoxyethylene/alkyl-co-modified organopolysiloxanes, linear polyoxypropylene/alkyl-co-modified organopolysiloxanes, and linear polyoxyethylene/polyoxypropylene/alkyl-co-modified organopolysiloxanes. Though not particularly limited, the chain length of alkyl is preferably 10 to 20 carbon atoms, more preferably 12 to 16 carbon atoms in view of compatibility. Most preferred is one known by the labeling name: cetyl PEG/PPG-10/1 dimethicone. It is commercially available, for example, under the tradename of KF-6048 (Shin-Etsu Chemical Co., Ltd.).

The amount of component (b) blended is 0.05 to 0.8% by weight, preferably 0.1 to 0.6% by weight, more preferably 0.2 to 0.5% by weight of the overall cosmetic composition. If the amount is less than 0.05% by weight, the cosmetic composition has low emulsion stability. If the amount exceeds 0.8% by weight, a water-breaking sensation is lost.

### [Component (c)]

Component (c) is an aqueous component which is not particularly limited as long as it is a component commonly used in cosmetics. The aqueous component is soluble in water at 25°C and forms a water phase in the W/O cosmetic composition. Examples include water, humectants, lower alcohols, water-soluble polymers, skin-beautifying ingredients, water-soluble inorganic salts, and pH adjusting agents, which may be used alone or in admixture of two or more.

Examples of water include water (labeling name, INCI name: Water), purified water (labeling name), distillates derived from fruits and plants, sea water (labeling name, INCI name: Sea Water), hot spring water (labeling name), and peat water (labeling name, INCI name: Peat Water), which are commonly used in cosmetics. The content of water blended is preferably 5 to 85% by weight of component (c). A water content of at least 5% by weight ensures a more water-breaking sensation whereas a water content of up to 85% by weight ensures a better feeling on use.

Suitable humectants include sugar alcohols such as sorbitol (labeling name, INCI name: Sorbitol), maltose (labeling name, INCI name: Maltose), xylitol (labeling name, INCI name: Xylitol); polyhydric alcohols such as BG (labeling name, INCI name: Butylene Glycol), DPG (labeling name, INCI name: Dipropylene Glycol), pentylene glycol (labeling name, INCI name: Pentylene Glycol), 1,10-decanediol (labeling name, INCI name: 1,10-Decanediol), octanediol (labeling name, INCI name: Octanediol), hexanediol (labeling name, INCI name: Hexanediol), erythritol (labeling name, INCI name: Erythritol), glycerin (labeling name, INCI name: Glycerin), diglycerin (labeling name, INCI name: Diglycerin), and polyethylene glycol; glucose (labeling name, INCI name: Glucose), glyceryl glucoside (labeling name, INCI name: Glyceryl Glucoside), betaine (labeling name, INCI name: Betaine), and sodium chondroitin sulfate (labeling name, INCI name: Sodium Chondroitin Sulfate). The content of humectant is preferably 5 to 70% by weight, more preferably 10 to 30% by weight of component (c). Blending of the humectant enables the cosmetic composition to improve its emulsion stability and to adjust its water-breaking sensation.

Suitable lower alcohols include ethanol (labeling name, INCI name: Ethanol) and isopropanol (labeling name, INCI name: Isopropyl Alcohol).

Suitable water-soluble polymers include naturally occurring water-soluble polymers such as carrageenan (labeling name, INCI name: Carrageenan), sodium hyaluronate (labeling name, INCI name: Sodium Hyaluronate), potassium hyaluronate (labeling name, INCI name: Potassium Hyaluronate), and xanthan gum (labeling name, INCI name: Xanthan Gum); semi-synthetic water-soluble polymers such as hydroxyethyl cellulose (labeling name, INCI name: Hydroxyethylcellulose), hydroxypropyl methylcellulose (labeling name, INCI name: Hydroxypropyl Methylcellulose), and calcium carboxymethyl cellulose (labeling name, INCI name: Calcium Carboxymethyl Cellulose); synthetic water-soluble polymers such as polyvinyl alcohol (labeling name, INCI name: Polyvinyl Alcohol) and carbomer (labeling name, INCI name: Carbomer); and inorganic water-soluble polymers such as bentonite (labeling name, INCI name: Bentonite).

Suitable skin-beautifying ingredients include whitening agents such as arbutin (labeling name, INCI name: Arbutin), ascorbic acid (labeling name, INCI name: Ascorbic Acid) and derivatives thereof; anti-inflammatory agents such as allantoin (labeling name, INCI name: Allantoin), dipotassium glycyrrhizinate (labeling name, INCI name: Dipotassium Glycyrrhizate), disodium glycyrrhizinate (labeling name, INCI name: Disodium Glycyrrhizate), potassium glycyrrhizinate (labeling name, INCI name: Potassium Glycyrrhizate), ammonium glycyrrhizinate (labeling name, INCI name: Ammonium Glycyrrhizate); and blood flow promoters such as benzyl nicotinate (labeling name, INCI name: Benzyl Nicotinate).

Other examples include water-soluble inorganic salts and pH adjusting agents such as sodium chloride (labeling name, INCI name: Sodium Chloride), magnesium sulfate (labeling name, INCI name: Magnesium Sulfate), sodium hydroxide (labeling name, INCI name: Sodium Hydroxide), potassium hydroxide (labeling name, INCI name: Potassium Hydroxide), arginine (labeling name, INCI name: Arginine), citric acid (labeling name, INCI name: Citric Acid), sodium citrate (labeling name, INCI name: Sodium Citrate), lactic acid (labeling name, INCI name: Lactic Acid), and glycolic acid (labeling name, INCI name: Glycolic Acid).

The amount of component (c) blended is 30 to 90% by weight, preferably 55 to 85% by weight, more preferably 60 to 85% by weight of the overall cosmetic composition. An amount of less than 30% by weight makes the composition stickier and gives little water-breaking sensation whereas an amount in excess of 90% by weight is detrimental to emulsion stability.

### [Component (d)]

Component (d) used herein is an oil component exclusive of silicone oil. The oil component is not particularly limited as long as it is a component commonly used in cosmetics. The oil component may be used alone or in admixture of two or more. The oil component is a component which does not dissolve in water at 25°C and forms an oil phase in the W/O cosmetic composition, with the silicone oil being excluded. The oil component may be either liquid or solid at 25°C. Among others, those oil components whose backbone is composed of hydrocarbon chain are preferred. Examples of the oil component whose backbone is composed of hydrocarbon chain include hydrocarbon oils, ester oils, plant oils, solid or semi-solid oily components, and fluorochemical oils.

Suitable hydrocarbon oils include straight or branched hydrocarbon oils and may be either volatile or non-volatile hydrocarbon oils. Examples include olefin oligomer (labeling name), isododecane (labeling name, INCI name: Isododecane), dodecane (labeling name, INCI name: Dodecane), isohexadecane (labeling name, INCI name: Isohexadecane), undecane (labeling name, INCI name: Undecane), squalane (labeling name, INCI name: Squalane), squalene (labeling name, INCI name: Squalene), mineral oil (labeling name, INCI name: Mineral Oil), liquid isoparaffin (labeling name), polyisobutylene (labeling name), hydrogenated polyisobutylene (labeling name, INCI name: Hydrogenated Polyisobutylene), C13-15 alkane (labeling name, INCI name: C13-15 Alkane), and mineral oil (labeling name, INCI name: Mineral Oil). Of these, squalane (labeling name, INCI name: Squalane) and mineral oil (labeling name, INCI name: Mineral Oil) are preferred because a moist feeling on use is available. These hydrocarbon oils are liquid at 25°C and preferably have a kinematic viscosity of 1 to 60 mm²/s, more preferably 1 to 40 mm²/s. As used herein, the kinematic viscosity is measured at 25°C by an Ostwald viscometer. The unit "mm²/s" is sometimes designated as "cSt."

Suitable ester oils include low polar ester oils and high polar ester oils. The low polar ester oils are ester oils having an inorganic-organic balance (IOB) value of less than 0.3 whereas the high polar ester oils are ester oils having an IOB value of at least 0.3. The IOB value is computed from a ratio of inorganic value to organic value, i.e., IV/OV in the organic conception diagram. For the detail of organic conception diagram, reference should be made to Yoshio Koda, "Organic Conception Diagram --Fundamental and Application--," Sankyo Publishing, 1984. Examples of the low polar oil include C12-15 alkyl benzoate (labeling name, INCI name: C12-15 Alkyl Benzoate) with IOB = 0.18, isodecyl isononanoate (labeling name, INCI name: Isotridecyl Isononanoate) with IOB = 0.16, isnonyl isononanoate (labeling name, INCI name: Isononyl Isononanoate) with IOB = 0.2, cetyl ethylhexanoate (labeling name, INCI name: Cetyl Ethylhexanoate) with IOB = 0.13, ethylhexyl palmitate (labeling name, INCI name: Ethylhexyl Isopalmitate) with IOB = 0.13, isopropyl myristate (labeling name, INCI name: Isopropyl Myristate) with IOB = 0.18, and coco-alkyl caprylate/caprate (labeling name, INCI name: Coco-Caprylate/Caprate) with IOB = 0.15. Examples of the high polar oil include triethylhexanoin (labeling name, INCI name: Triethylhexanoin) with IOB = 0.35, caprylic/capric triglyceride (labeling name, INCI name: Caprylic/Capric Triglyceride) with IOB = 0.3, and neopentyl glycol diethylhexanoate (labeling name, INCI name: Neopentyl Glycol Diethylhexanoate) with IOB = 0.32. These ester oils are liquid at 25°C.

Suitable plant oils include those among oils other than the aforementioned hydrocarbon oils and ester oils, such as coconut oil (labeling name, INCI name: Cocos Nucifera (Coconut) Oil), hydrogenated coconut oil (labeling name, INCI name: Hydrogenated Coconut Oil), avocado oil (labeling name, INCI name: Persea Gratissima (Avocado) Oil), olive oil (labeling name, INCI name: Olea Europaea (Olive) Fruit Oil), apricot kernel oil (labeling name, INCI name: Prunus Armeniaca (Apricot) Kernel Oil), kukui nut oil (labeling name, INCI name: Aleurites Moluccana Seed Oil), grape seed oil (labeling name, INCI name: Vitis Vinifera (Grape) Seed Oil), safflower oil (labeling name, INCI name: Carthamus Tinctorius (Safflower) Seed Oil), almond oil (labeling name, INCI name: Prunus Amygdalus Dulcis (Sweet Almond) Oil), corn germ oil (labeling name, INCI name: Zea Mays (Corn) Germ Oil), sunflower oil (labeling name, INCI name: Helianthus Annuus (Sunflower) Seed Oil), hazel nut oil (labeling name, INCI name: Gevuina Avellana Seed Oil), jojoba seed oil (labeling name, INCI name: Simmondsia Chinensis (Jojoba) Seed Oil), macadamia nut oil (labeling name, INCI name: Macadamia Ternifolia Seed Oil), meadowfoam oil (labeling name, INCI name: Limnanthes Alba (Meadowfoam) Seed Oil), and dog rose hips oil (labeling name, INCI name: Rosa Canina Fruit Oil).

Suitable solid oil components include those which are solid at 25°C, among oils other than the aforementioned hydrocarbon oils, ester oils and plant oils. They are not particularly limited as long as they are raw materials commonly used in cosmetics. Examples include plant waxes such as carnauba wax (labeling name, INCI name: Copernicia Cerifera (Carnauba) Wax), candelilla wax (labeling name, INCI name: Euphorbia Cerifera (Candelilla) Wax), and shea butter (labeling name, INCI name: Butyrospermum Parkii (Shea) Butter); animal waxes such as beeswax (labeling name, INCI name: Beeswax); hydrocarbon waxes such as synthetic wax (labeling name, INCI name: Synthetic Wax), solid paraffin (labeling name, INCI name: Paraffin), polyethylene (labeling name, INCI name: Polyethylene), ceresin (labeling name, INCI name: Ceresin), microcrystalline wax (labeling name, INCI name: Microcrystalline Wax); higher alcohols such as stearyl alcohol (labeling name, INCI name: Stearyl Alcohol), behenyl alcohol (labeling name, INCI name: Behenyl Alcohol) and cetanol (labeling name, INCI name: Cetyl Alcohol); fatty acids such as stearic acid (labeling name, INCI name: Stearic Acid) and behenic acid (labeling name, INCI name: Behenic Acid); and derivatives thereof. One or more compounds selected from the foregoing are preferred.

Suitable semi-solid oil components include those which are pasty at 25°C among oils other than the aforementioned hydrocarbon oils, ester oils and plant oils. They are not particularly limited as long as they are raw materials commonly used in cosmetics. Examples include lanolin (labeling name, INCI name: Lanolin), vaseline (labeling name, INCI name: Petrolatum), dipentaerythritol fatty acid esters, and hardened or hydrogenated oil. One or more compounds selected from the foregoing are preferred.

Suitable fluorochemical oils include perfluoropolyether, perfluorodecalin and perfluorooctane.

The amount of component (d) blended is 5 to 30% by weight, preferably 8 to 20% by weight, more preferably 10 to 15% by weight of the overall cosmetic composition.

The content of the hydrocarbon oil and low polar ester oil is preferably at least 80% by weight of component (d) in view of compatibility with components (a) and (b), more preferably at least 90% by weight, most preferably 100% by weight of component (d). A content of at least 80% by weight ensures a further improvement in emulsion stability.

### [Component (e)]

Component (e) is an alkyl-modified, partially crosslinked organopolysiloxane exclusive of component (a), which may be used alone or in admixture. Blending of component (e) enables the cosmetic composition to improve its emulsion stability.

The alkyl-modified, partially crosslinked organopolysiloxane is a three-dimensional crosslinked product obtained by crosslinking an organopolysiloxane chain having alkyl-modifying chains on its backbone with organopolysiloxane chains. Exemplary of the alkyl-modified, partially crosslinked organopolysiloxane is vinyl dimethicone/lauryl dimethicone crosspolymer (labeling name, INCI name: Vinyl Dimethicone/Lauryl Dimethicone Crosspolymer). Preferred as component (e) are swollen products which are previously swollen in oils such as hydrocarbon oils. Specifically, they are commercially available as swollen products, typically gels, containing hydrocarbon oils or other oils, for example, under the tradename of KSG-41A, 42A, 43, and 44 (all from Shin-Etsu Chemical Co., Ltd.). Exemplary hydrocarbon oils include those described above for component (d).

When used, the amount of component (e) blended is 0.1 to 2% by weight, preferably 0.2 to 0.8% by weight as solids of the overall cosmetic composition. An amount of at least 0.1% by weight ensures better stability whereas an amount of up to 2% by weight ensures improvements in moistness and feeling on use during application.

Various components which are commonly used in cosmetics may be blended in the cosmetic composition as long as the benefits of the invention are not impaired. Suitable components include (1) an oil other than component (d), (2) a powder, (3) an oil-soluble gelling agent, (4) a film-forming agent, (5) a surfactant other than components (a) and (b), and (6) other optional additives. These components may be used alone or in a suitable combination. Any of these components may be selected and used depending on a cosmetic composition of particular type while the amount thereof may be any well-known amount depending on a cosmetic composition of particular type.

### (1) Oil other than component (d)

The oil is not particularly limited as long as it is other than component (d). It may be solid, semi-solid or liquid at room temperature. Any suitable oil, for example, silicone oil may be blended for the purpose of adjusting a feeling on use. When used, the amount of oil blended is preferably 0.1 to 10% by weight, more preferably 0.1 to 5% by weight of the overall cosmetic composition.

The silicone oil is not particularly limited as long as it is a raw material commonly used in cosmetics. Examples include linear or branched organopolysiloxanes having a viscosity of 1 to 20 mm²/s at 25°C such as dimethylpolysiloxane, cyclopentasiloxane, cyclohexasiloxane, disiloxane, trisiloxane, methyltrimethicone, methylphenylpolysiloxane, methylhexylpolysiloxane, methylhydrogenpolysiloxane, diphenylsiloxyphenyltrimethicone (labeling name), and dimethylsiloxane-methylphenylsiloxane copolymers; alkyl-modified silicones such as caprylyl methicone, amino-modified organopolysiloxane, silicone rubbers such as gum-like dimethylpolysiloxane having a high degree of polymerization, gum-like amino-modified organopolysiloxane, and gum-like dimethylsiloxane-methylphenylsiloxane copolymers, cyclic siloxane solutions of silicone gum or rubber, higher alkoxy-modified organopolysiloxanes such as stearoxysilicone, higher fatty acid-modified organopolysiloxanes, fluorine-modified organopolysiloxanes, silicone resins, and solutions of silicone resins, and acrylate/dimethicone copolymers. Of these, volatile silicones capable of providing a light feeling are preferably used, which are commercially available as TMF-1.5, KF-995, and KF-96A-2cs from Shin-Etsu Chemical Co., Ltd. More particularly, phenylsilicones such as diphenylsiloxyphenyl trimethicone which are employed for the purposes of improving compatibility with component (d) and lustering are more preferably used, which are commercially available as KF-56A from Shin-Etsu Chemical Co., Ltd.

A stable emulsion is obtained when component (d) accounts for a large proportion of the total amount of components (d) and (1). The large proportion means that the amount of component (d) blended is at least 80% by weight of the total amount of components (d) and (1). A stable emulsion is obtained particularly when the amount of component (d) is at least 90% by weight and even 100% by weight.

### (2) Powder

The powder used herein is not particularly limited as long as it is a raw material commonly used in cosmetics. For example, silicone spherical particle powder, coloring pigments, and UV scattering agents may be used. When used, the amount of powder blended is preferably 0.1 to 20% by weight, more preferably 1 to 10% by weight of the overall cosmetic composition.

### • Silicone spherical particle powder

Suitable silicone spherical particle powders include crosslinked silicone spherical particle powder (i.e., so-called silicone rubber powder consisting of organopolysiloxane of the crosslinked structure having a chain of repeating diorganosiloxane units), and silicone resin particles (polyorganosilsesquioxane resin particles of three-dimensional network structure). Examples are known under the name of (dimethicone/vinyl dimethicone) crosspolymer and polymethylsilsesquioxane. They are commercially available in spherical powder form, for example, under the tradename of KMP-598, 590, 591, and 592, or commercially available as swollen products containing silicone oil, for example, under the tradename of KSG-016F (all from Shin-Etsu Chemical Co., Ltd.). These powders may be used alone or in admixture of two or more.

In particular, silicone resin-coated silicone rubber powder is applied in sunscreen, make-up and concealer products because of its feel-improving effect (sticky feel prevention) and morphological correcting effect for wrinkles and pores. Examples of the silicone resin-coated silicone rubber powder include (vinyl dimethicone/methicone silsesquioxane) crosspolymer, (diphenyl dimethicone/vinyldiphenyl dimethicone/silsesquioxane) crosspolymer, polysilicone-22, and polysilicone-1 crosspolymer, as defined in the Japanese labeling name. They are commercially available, for example, under the tradename of KSP-100, 101, 102, 105, 300, 411, and 441 (all from Shin-Etsu Chemical Co., Ltd.). These powders may be used alone or in admixture of two or more.

### • Coloring pigment

The coloring pigment used herein is not particularly limited as long as it is commonly used in make-up cosmetics. Examples include inorganic pigments such as talc, mica, kaolin, silica, calcium carbonate, zinc white, titanium oxide (titania), red iron oxide, yellow iron oxide, black iron oxide, ultramarine, Prussian blue, carbon black, low-order titanium oxide, cobalt violet, chromium oxide, chromium hydroxide, cobalt titanate, bismuth oxychloride, and titanium-mica base pearl pigment; organic pigments in the form of zirconium, barium or aluminum lakes such as Red #201, Red #202, Red #204, Red #205, Red #220, Red #226, Red #228, Red #405, Orange #203, Yellow #205, Yellow #4, Yellow #5, Blue #1, Blue #404, and Green #3; natural coloring agents such as chlorophyll and β-carotene; and dyes. The pigments which are surface-treated with silicone or the like are also useful. The silicone-treated coloring pigment is commercially available, for example, under the tradename of KTP-09W, 09R, 09Y and 09B (all from Shin-Etsu Chemical Co., Ltd.).

### • UV scattering agent

The UV scattering agent used herein is not particularly limited as long as it is a raw material commonly blended in cosmetics. Examples include titanium oxide, zinc oxide, and cerium oxide. Unlike the pigment, the UV scattering agent preferably has an average primary particle size of up to 200 nm. The UV scattering agent which is hydrophobized with silicone or the like is also useful. Examples of the hydrophobized inorganic powder include hydrophobized microparticulate titanium oxide and hydrophobized microparticulate zinc oxide, which are commercially available as dispersions, for example, under the tradename of SPD-T5, T6, T5L, Z5, Z6, and Z5L (all from Shin-Etsu Chemical Co., Ltd.).

### • Partially crosslinked organopolysiloxane exclusive of components (a) and (e)

The partially crosslinked organopolysiloxane exclusive of components (a) and (e) is not particularly limited as long as it is a raw material commonly blended in cosmetics. Examples include (vinyl dimethicone/dimethicone) crosspolymer and (dimethicone/phenyl vinyl dimethicone) crosspolymer, as defined in the labeling name. They may be used alone or in admixture. They are commercially available as swollen products, typically gel, containing silicone oil or another oil, for example, under the tradename of KSG-15, 16, 19 and 18A (all from Shin-Etsu Chemical Co., Ltd.). Inter alia, KSG-18A is most preferred for compatibility.

### (3) Oil-soluble gelling agent

The oil-soluble gelling agent is not particularly limited as long as it is a raw material commonly blended in cosmetics. Suitable gelling agents include waxes, sugar fatty acid esters, and organo-modified clay minerals.

Examples of the sugar fatty acid ester include dextrin fatty acid esters such as dextrin palmitate, dextrin stearate, and dextrin 2-ethylhexanate palmitate; sucrose fatty acid esters such as sucrose palmitate and sucrose stearate; fructooligosaccharide fatty acid esters such as fructooligosaccharide stearate and fructooligosaccharide 2-ethylhexanoate. Also included are organo-modified clay minerals such as disteardimonium hectorite, stearalkonium hectorite and hectorite.

### (4) Film-forming agent

The film-forming agent is not particularly limited as long as it is a raw material commonly used in cosmetics. Examples include latexes such as polyvinylalcohol, polyvinylpyrrolidone, polyvinylacetate, polyalkylacrylates; cellulose derivatives such as dextrin, alkyl celluloses, and nitrocellulose; silicone-modified polysaccharides such as pullulan tri(trimethylsiloxy)silylpropylcarbamate, silicone-modified polyvinylalcohols such as (butenediol/vinyl alcohol) tri(trimethylsiloxy)silylpropylcarbamate copolymers, acrylic silicone base graft copolymers such as (alkyl acrylate/dimethicone) copolymers, silicone resins such as trimethylsiloxysilicic acid, silicone base resins such as silicone-modified polynorbornene and fluorine-modified silicone resins, fluoro-resins, aromatic hydrocarbon resins, polymer emulsion resins, terpene resins, polybutene, polyisoprene, alkyd resins, polyvinylpyrrolidone-modified polymers, rosin-modified resins, and polyurethane.

Among these, silicone base film-forming agents are preferred in view of long lasting performance. More preferred examples include, but are not limited to, pullulan tri(trimethylsiloxy)silylpropylcarbamate which is commercially available in solvent solution form as TSPL-30-D5, ID from Shin-Etsu Chemical Co., Ltd., (alkyl acrylate/dimethicone) copolymers which are commercially available in solvent solution form as KP-543, 545, 549, 550, and 545L from Shin-Etsu Chemical Co., Ltd., trimethylsiloxysilicic acid which is commercially available in solvent solution form as KF-7312J and X-21-5250 from Shin-Etsu Chemical Co., Ltd., and silicone-modified polynorbornene which is commercially available in solvent solution form as NBN-30-ID from Shin-Etsu Chemical Co., Ltd.. The film-forming agent may be used alone or in admixture.

### (5) Surfactant

Any surfactants other than components (a) and (b) may be used. Suitable surfactants include nonionic, anionic, cationic and ampholytic surfactants, any of which may be used. The surfactant used herein is not particularly limited as long as it is commonly used in cosmetics and the benefits of the invention are not impaired. Commercial products include, for example, KF-6011, KF-6011P, KF-6043, KF-6012, KF-6013, KF-6015, KF-6016, KF-6017, KF-6028, KF-6028P, KF-6038, KF-6100, KF-6104, KF-6105, KF-6106, KSG-210, KSG-240, and KSG-710 (all from Shin-Etsu Chemical Co., Ltd.).

### (6) UV absorber

The UV absorber is not particularly limited as long as it is a raw material commonly blended in cosmetics. Examples include homomenthyl salicylate, octocrylene, 4-tert-butyl-4'-methoxydibenzoylmethane, 4-(2-β-glucopyranosiloxy)propoxy-2-hydroxybenzophenone, octyl salicylate, hexyl 2-[4-(diethylamino)-2-hydroxybenzoyl]benzoate, dihydroxydimethoxybenzophenone, sodium dihydroxydimethoxybenzophenonedisulfonate, dihydroxybenzophenone, dimethicodiethylbenzal malonate, 1-(3,4-dimethoxyphenyl)-4,4-dimethyl-1,3-pentanedione, 2-ethylhexyl dimethoxybenzylidenedioxoimidazolidinepropionate, tetrahydroxybenzophenone, terephthalylidene dicamphor sulfonic acid, 2,4,6-tris[4-(2-ethylhexyloxycarbonyl)anilino]-1,3,5-triazine, methylbis(trimethylsiloxy)silylisopentyl trimethoxycinnamate, drometrizole trisiloxane, 2-ethylhexyl p-dimethylaminobenzoate, isopropyl p-methoxycinnamate, 2-ethylhexyl p-methoxycinnamate, 2,4-bis[{4-(2-ethylhexyloxy)-2-hydroxy}-phenyl]-6-(4-methoxyphenyl)-1,3,5-triazine, 2-hydroxy-4-methoxybenzophenone, hydroxymethoxybenzophenone sulfonic acid and trihydrate thereof, sodium hydroxymethoxybenzophenone sulfonate, phenylbenzimidazole sulfonic acid, and 2,2'-methylenebis[6-(2H-benzotriazol-2-yl)-4-(1,1,3,3-tetramethylbutyl)phenol]. Also, a UVA absorber, e.g., hexyl diethylaminohydroxybenzoylbenzoate may be combined with a UVB absorber, e.g., ethylhexyl methoxycinnamate. Any two or more of the foregoing may be used in combination.

### (7) Other optional components

Other additives include oil-soluble preservatives, bactericides, anti-inflammatory agents, and perfumes.

### [Preparation method]

The W/O cosmetic composition of the invention may be prepared by any well-known methods, for example, a method involving the steps of mixing an oil phase composition containing the aforementioned components with a water phase composition, and emulsifying them by means of a puddle, disperser or homogenizer.

### [W/O cosmetic composition]

The cosmetic composition is of water-in-oil (W/O) type and may be viscous or solid. In the case of a viscous composition, its viscosity is preferably in the range of 10,000 to 200,000 mPa·s, more preferably 20,000 to 100,000 mPa·s, even more preferably 30,000 to 100,000 mPa·s. Notably, the viscosity is measured by a Brookfield viscometer, for example, DV-III Ultra Rheometer (Brookfield Engineering Laboratories Inc.) with spindle T-F while moving the spindle downward at a rate of 2.2 cm/min.

The emulsified state may be either W/O emulsion or O/W/O emulsion. When applied to the skin, the cosmetic composition of water-breaking type provides a sensation or feeling that the internal water phase bursts out. As used herein, the term "water break" refers to the phenomenon that shearing forces acting on a cosmetic preparation upon application decompose or break the W/O emulsion whereby the water phase or internal phase bursts out as watery droplets. Such feeling or sensation is achieved by stabilizing a cosmetic composition in which the diameter of emulsified droplets is large or irregular as observed under a microscope. Specifically, the diameter of emulsified droplets is 1 to 30 µm, preferably 2 to 20 µm as most often observed. It is noted that the diameter of emulsified droplets is measured by placing 3 µL of a test sample on a slide glass, placing a cover glass thereon to form a specimen, observing the specimen under an optical microscope, measuring the diameter of emulsified droplets, and calculating an arithmetic average diameter of 20 droplets.

The type of the cosmetic composition is not particularly limited as long as it contains essential components. For example, the cosmetic composition may be implemented as toilet water, lotion, milky lotion, cream, massage cream, hair care, foundation, foundation primer, eyeliner, or other products. The cosmetic composition may take a variety of formulas including liquid, cream, solid, gel, mousse, stick, etc.

### EXAMPLES

Examples and Comparative Examples are shown below for further illustrating the invention although the invention is not limited thereto. In Examples, compositional percent (%) is by weight unless otherwise stated. The amount is the amount of a component in the designated product in which it is blended.

### [Example 1]

### [Examples 1 to 13 and Comparative Examples 1 to 9]

W/O cosmetic compositions of the formulation shown in Tables 2 to 6 were prepared by the following procedure and evaluated by the following tests.

### <Preparation of cosmetic compositions of Examples 1 to 13 and Comparative Examples 1 to 9>

A W/O cosmetic composition was prepared by the steps:
A: mixing part (1) uniformly,
B: mixing part (2) uniformly, and
C: adding B to A, followed by emulsification.

### (1) Emulsion stability

A 30-mL vial was filled with the cosmetic composition and stored in a thermostatic chamber at 50°C for 1 month, after which the viscosity of the composition was measured.

In terms of a change of viscosity from that immediately after preparation, the rating is excellent "⊚" for a change of 0% to less than 25%, good "O" for a change of 25% to less than 40%, fair "Δ" for a change of 40% to less than 50%, and poor "×" for a change of 50% or more or separated. The composition which was rated poor "×" in stability was no longer evaluated for properties. The viscosity of the compositions of Examples and Comparative Examples immediately after preparation was in the range of 10,000 to 200,000 mPa·s when they were viscous.

The viscosity was measured by DV-III Ultra Rheometer (Brookfield Engineering Laboratories Inc.) with spindle T-F at a spindle downward movement of 2.2 cm/min, a rotational speed of 6 rpm, and a time of 60 seconds.

A sample is judged Pass for "Δ" or above.

### (2) Evaluation of properties

The cosmetic compositions were evaluated in terms of water-breaking sensation (or bursting of watery droplets), spread (or extensibility), and feel-on-use (or non-sticky feel) by a panel of ten members according to the evaluation criteria in Table 1. The result is an average of ratings of 10 panel members and rated according to the following judgment criteria. The results are also shown in Tables.

**[Table 1]**

| Points | Water-breaking sensation | Spread | Feel-on-use |
|---|---|---|---|
| 5 | droplets burst out | excellent | non-sticky |
| 4 | - | good | less sticky |
| 3 | few droplets burst out | mediocre | mediocre |
| 2 | - | rather poor | rather sticky |
| 1 | no droplets burst out | poor | sticky |

### Judgment criteria

⊚: average point ≥ 4.5
⊚: 3.5 ≤ average point < 4.5
Δ: 2.5 ≤ average point < 3.5
×: 1.5 ≤ average point < 2.5
× ×: average point < 1.5

A sample is judged Pass for "Δ" or above.

**[Table 2]**

| Composition (%) | | Example | | | | |
|---|---|---|---|---|---|---|
| | | 1 | 2 | 3 | 4 | 5 |
| (1) | (a) KSG-310 ^{∗}1 | 3 | 3 | 3 | 3 | 3 |
| | (b) KF-6048 ^{∗}6 | 0.2 | 0.2 | 0.2 | 0.2 | 0.2 |
| | (e) KSG-44 ^{∗}7 | - | 1 | 1 | 1 | 1 |
| | (d) squalane | 11.8 | 10.8 | | | 8 |
| | (d) isotridecyl isononanoate | | | 10.8 | | |
| | (d) triethylhexanoin | | | | 10.8 | 2.8 |
| (2) | BG | 8 | 8 | 8 | 8 | 8 |
| | ethanol | 5 | 5 | 5 | 5 | 5 |
| | sodium chloride | 0.5 | 0.5 | 0.5 | 0.5 | 0.5 |
| | sodium citrate | 0.2 | 0.2 | 0.2 | 0.2 | 0.2 |
| | purified water | balance | balance | balance | balance | balance |
| Total | | 100 | 100 | 100 | 100 | 100 |
| (c) aqueous component | | 85 | 85 | 85 | 85 | 85 |
| Evaluation | Emulsion stability | 0 | ⊚ | O | Δ | O |
| | Water break sensation | ⊚ | ⊚ | ⊚ | ⊚ | ⊚ |
| | Spread | ⊚ | ⊚ | O | O | O |
| | Feel-on-use | ⊚ | ⊚ | O | Δ | Δ |

| | | | | | | |
|---|---|---|---|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}6 cetyl PEG/PPG-10/1 dimethicone (labeling name, INCI name: Cetyl PEG/PPG-10/1 Dimethicone) ^{∗}7 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) | | | | | | |

**[Table 3]**

| Composition (%) | | Example | | | |
|---|---|---|---|---|---|
| | | 6 | 7 | 8 | 9 |
| (1) | (a) KSG-310 ^{∗}1 | | | | |
| | (a) KSG-330 ^{∗}2 | 3 | | | |
| | (a) KSG-810 ^{∗}3 | | 3 | | |
| | (a) KSG-320Z ^{∗}4 | | | 3 | |
| | (a) KSG-820Z ^{∗}5 | | | | 3 |
| | (b) KF-6048 ^{∗}6 | 0.2 | 0.2 | 0.2 | 0.2 |
| | (e) KSG-44 ^{∗}7 | 1 | 1 | 1 | 1 |
| | (d) squalane | 10.8 | 10.8 | 10.8 | 10.8 |
| (2) | BG | 8 | 8 | 8 | 8 |
| | ethanol | 5 | 5 | 5 | 5 |
| | sodium chloride | 0.5 | 0.5 | 0.5 | 0.5 |
| | sodium citrate | 0.2 | 0.2 | 0.2 | 0.2 |
| | purified water | balance | balance | balance | balance |
| Total | | 100 | 100 | 100 | 100 |
| (c) aqueous component | | 85 | 85 | 85 | 85 |
| Evaluation | Emulsion stability | Δ | Δ | Δ | Δ |
| | Water break sensation | ⊚ | ⊚ | ⊚ | ⊚ |
| | Spread | O | O | O | O |
| | Feel-on-use | O | O | O | O |

| | | | | | |
|---|---|---|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}2 mixture of 20% (PEG-15/lauryl dimethicone) crosspolymer (labeling name, INCI name: PEG-15/Lauryl Dimethicone Crosspolymer) and 80% triethylhexanoin (labeling name, INCI name: Triethylhexanoin) ^{∗}3 mixture of 30% (lauryl dimethicone/polyglycerin-3) crosspolymer (labeling name, INCI name: Lauryl Dimethicone/Polyglycerin-3 Crosspolymer) and 70% mineral oil (labeling name, INCI name: Mineral Oil) ^{∗}4 mixture of 25% (PEG-15/lauryl polydimethylsiloxyethyl dimethicone) crosspolymer (labeling name, INCI name: PEG-15/Lauryl Polydimethylsiloxyethyl Dimethicone Crosspolymer) and 75% isododecane (labeling name, INCI name: Isododecane) ^{∗}5 mixture of 25% (lauryl dimethicone/polyglycerin-3) crosspolymer (labeling name, INCI name: Lauryl Dimethicone/Polyglycerin-3 Crosspolymer) and 75% isododecane (labeling name, INCI name: Isododecane) ^{∗}6 cetyl PEG/PPG-10/1 dimethicone (labeling name, INCI name: Cetyl PEG/PPG-10/1 Dimethicone) ^{∗}7 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) | | | | | |

**[Table 4]**

| Composition (%) | | Example | | | |
|---|---|---|---|---|---|
| | | 10 | 11 | 12 | 13 |
| (1) | (a) KSG-310 ^{∗}1 | 3 | 3 | 3 | 3 |
| | (b) KF-6048 ^{∗}2 | 0.05 | 0.1 | 0.6 | 0.8 |
| | (e) KSG-44 ^{∗}3 | 1 | 1 | 1 | 1 |
| | (d) squalane | 11 | 10.9 | 10.5 | 10.2 |
| (2) | BG | 8 | 8 | 8 | 8 |
| | ethanol | 5 | 5 | 5 | 5 |
| | sodium chloride | 0.5 | 0.5 | 0.5 | 0.5 |
| | sodium citrate | 0.2 | 0.2 | 0.2 | 0.2 |
| | purified water | balance | balance | balance | balance |
| Total | | 100 | 100 | 100 | 100 |
| (c) aqueous component | | 85 | 85 | 85 | 85 |
| Evaluation | Emulsion stability | Δ | O | ⊚ | ⊚ |
| | Water break sensation | ⊚ | ⊚ | O | Δ |
| | Spread | Δ | Δ | ⊚ | O |
| | Feel-on-use | ⊚ | ⊚ | ⊚ | O |

| | | | | | |
|---|---|---|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}2 cetyl PEG/PPG-10/1 dimethicone (labeling name, INCI name: Cetyl PEG/PPG-10/1 Dimethicone) ^{∗}3 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) | | | | | |

**[Table 5]**

| Composition (%) | | Comparative Example | | | | |
|---|---|---|---|---|---|---|
| | | 1 | 2 | 3 | 4 | 5 |
| (1) | (a) KSG-310 ^{∗}1 | | 3 | | 3 | 3 |
| | KSG-210 ^{∗}2 | | | 3 | | |
| | (b) KF-6048 ^{∗}3 | 0.2 | | 0.2 | | |
| | KF-6038 ^{∗}4 | | | | 0.2 | |
| | KF-6105 ^{∗}5 | | | | | 0.2 |
| | (e) KSG-44 ^{∗}6 | 1 | 1 | 1 | 1 | 1 |
| | (d) squalane | 13.8 | 11 | 10.8 | 10.8 | 10.8 |
| (2) | BG | 8 | 8 | 8 | 8 | 8 |
| | ethanol | 5 | 5 | 5 | 5 | 5 |
| | sodium chloride | 0.5 | 0.5 | 0.5 | 0.5 | 0.5 |
| | sodium citrate | 0.2 | 0.2 | 0.2 | 0.2 | 0.2 |
| | purified water | balance | balance | balance | balance | balance |
| Total | | 100 | 100 | 100 | 100 | 100 |
| (c) aqueous component | | 85 | 85 | 85 | 85 | 85 |
| Evaluation | Emulsion stability | × | × | × | × | × |

| | | | | | | |
|---|---|---|---|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}2 mixture of 25% (dimethicone/(PEG-10/15)) crosspolymer (labeling name, INCI name: Dimethicone/(PEG-10/15) Crosspolymer and 75% dimethicone (labeling name, INCI name: Dimethicone) ^{∗}3 cetyl PEG/PPG-10/1 dimethicone (labeling name, INCI name: Cetyl PEG/PPG-10/1 Dimethicone) ^{∗}4 lauryl PEG-9 polydimethylsiloxyethyl dimethicone (labeling name, INCI name: Lauryl PEG-9 Polydimethylsiloxyethyl Dimethicone) ^{∗}5 lauryl polyglyceryl-3 polydimethylsiloxyethyl dimethicone (labeling name, INCI name: Lauryl Polyglyceryl-3 Polydimethylsiloxyethyl Dimethicone) ^{∗}6 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) | | | | | | |

**[Table 6]**

| Composition (%) | | Comparative Example | | | |
|---|---|---|---|---|---|
| | | 6 | 7 | 8 | 9 |
| (1) | (a) KSG-310 ^{∗}1 | 3 | 3 | 3 | 3 |
| | (b) KF-6048 ^{∗}2 | 1.0 | 0.2 | 0.2 | 0.2 |
| | (e) KSG-44 ^{∗}3 | 1 | 1 | 1 | 1 |
| | (d) squalane | 10 | 75.8 | 2.8 | |
| | dimethicone ^{∗}4 | | | | 10.8 |
| (2) | BG | 8 | 8 | 8 | 8 |
| | ethanol | 5 | 5 | 5 | 5 |
| | sodium chloride | 0.5 | 0.5 | 0.5 | 0.5 |
| | sodium citrate | 0.2 | 0.2 | 0.2 | 0.2 |
| | purified water | balance | balance | balance | balance |
| Total | | 100 | 100 | 100 | 100 |
| (c) aqueous component | | 85 | 20 | 93 | 85 |
| Evaluation | Emulsion stability | ⊚ | × | × | × |
| | Water break sensation | × | - | - | - |
| | Spread | ⊚ | - | - | - |
| | Feel-on-use | × | - | - | - |

| | | | | | |
|---|---|---|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}2 cetyl PEG/PPG-10/1 dimethicone (labeling name, INCI name: Cetyl PEG/PPG-10/1 Dimethicone) ^{∗}3 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) ^{∗}4 dimethicone (labeling name, INCI name: Dimethicone) | | | | | |

As seen from the results in Tables 2 to 6, the W/O cosmetic compositions of Examples 1 to 13 are satisfactory in stability (50°C/1 month), water-breaking sensation (bursting of water droplets), spread (extensibility) and feel-on-use (non-stickiness). Comparative Examples 1 and 3 free of component (a), Comparative Examples 2, 4 and 5 free of component (b), Comparative Example 9 containing less than 5% by weight of component (d), Comparative Example 7 containing less than 30% by weight of component (c), and Comparative Example 8 containing more than 85% by weight of component (c) are unpreferable in stability. Comparative Example 6 containing more than 0.8% by weight of component (b) provides no water-breaking sensation and is strongly sticky.

### [Example 14]

### Hand cream

### <Preparation of cosmetic composition>

A hand cream was obtained by
A: uniformly mixing components 1 to 6,
B: uniformly mixing components 7 to 12,
C: adding B to A and emulsifying.

| | Formulation | (%) |
|---|---|---|
| 1. KSG-310 ^{∗}1 | | 3.0 |
| 2. KF-6048 ^{∗}2 | | 0.2 |
| 3. KSG-44 ^{∗}3 | | 1.0 |
| 4. squalane | | 8.0 |
| 5. vaseline | | 2.0 |
| 6. KP-550 ^{∗}4 | | 2.0 |
| 7. BG | | 8.0 |
| 8. sodium citrate | | 0.2 |
| 9. magnesium sulfate | | 0.3 |
| 10. phenoxyethanol | | 0.1 |
| 11. methylparaben | | 0.1 |
| 12. purified water | | balance |
| | Total | 100.0 |

| | | |
|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}2 cetyl PEG/PPG-10/1 dimethicone (labeling name, INCI name: Cetyl PEG/PPG-10/1 Dimethicone) ^{∗}3 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) ^{∗}4 mixture of 40% (acrylate/dimethicone) copolymer (labeling name, INCI name: Acrylates/Dimethicone Copolymer and 60% isododecane (labeling name, INCI name: Isododecane) | | |

The resulting hand cream was confirmed to show satisfactory water-breaking sensation, feel-on-use, and spread and have storage stability.

### [Example 15]

### Moisturizing cream

### <Preparation of cosmetic composition>

A moisturizing cream was obtained by
A: uniformly mixing components 1 to 6,
B: uniformly mixing components 7 to 13,
C: adding B to A and emulsifying.

| | Formulation | (%) |
|---|---|---|
| 1. KSG-840 ^{∗}1 | | 5.0 |
| 2. KF-6048 ^{∗}2 | | 0.5 |
| 3. KSG-44 ^{∗}3 | | 5.0 |
| 4. squalane | | 10.0 |
| 5. sunflower seed oil | | 0.5 |
| 6. macadamia seed oil | | 0.5 |
| 7. glycerin | | 5.0 |
| 8. sorbitol | | 0.5 |
| 9. glyceryl glucoside | | 0.5 |
| 10. phenoxyethanol | | 0.3 |
| 11. sodium citrate | | 0.2 |
| 12. sodium chloride | | 0.5 |
| 13. purified water | | balance |
| | Total | 100.0 |

| | | |
|---|---|---|
| ^{∗}1 mixture of 30% (lauryl dimethicone/polyglycerin-3) crosspolymer (labeling name, INCI name: Lauryl Dimethicone/Polyglycerin-3 Crosspolymer) and 70% squalane (labeling name, INCI name: Squalane) ^{∗}2 cetyl PEG/PPG-10/1 dimethicone (labeling name, INCI name: Cetyl PEG/PPG-10/1 Dimethicone) ^{∗}3 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) | | |

The resulting moisturizing cream was confirmed to show satisfactory water-breaking sensation, feel-on-use, and spread and have storage stability.

### [Example 16]

### Massage cream

### <Preparation of cosmetic composition>

A massage cream was obtained by
A: uniformly mixing components 1 to 7,
B: uniformly mixing components 8 to 12,
C: adding B to A and emulsifying.

| | Formulation | (%) |
|---|---|---|
| 1. KSG-340 ^{∗}1 | | 2.0 |
| 2. KSG-810 ^{∗}2 | | 2.0 |
| 3. KF-6048 ^{∗}3 | | 0.2 |
| 4. KSG-44 ^{∗}4 | | 1.0 |
| 5. mineral oil | | 13.6 |
| 6. dimethicone (6 cs) | | 1.4 |
| 7. KSP-441 ^{∗}5 | | 1.0 |
| 8. BG | | 8.0 |
| 9. phenoxyethanol | | 0.3 |
| 10. sodium citrate | | 0.2 |
| 11. sodium chloride | | 0.5 |
| 12. purified water | | balance |
| | Total | 100.0 |

| | | |
|---|---|---|
| ^{∗}1 mixture of 15% (PEG-15/lauryl dimethicone) crosspolymer (labeling name, INCI name: PEG-15/Lauryl Dimethicone Crosspolymer), 15% (PEG-10/lauryl dimethicone) crosspolymer (labeling name, INCI name: PEG-10/Lauryl Dimethicone Crosspolymer, and 70% squalane (labeling name, INCI name: Squalane) ^{∗}2 mixture of 30% (lauryl dimethicone/polyglycerin-3) crosspolymer (labeling name, INCI name: Lauryl Dimethicone/Polyglycerin-3 Crosspolymer) and 70% mineral oil (labeling name, INCI name: Mineral Oil) ^{∗}3 cetyl PEG/PPG-10/1 dimethicone (labeling name, INCI name: Cetyl PEG/PPG-10/1 Dimethicone) ^{∗}4 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) ^{∗}5 polysilicone-22 (labeling name, INCI name: Polysilicone-22) | | |

The resulting massage cream was confirmed to show satisfactory water-breaking sensation, feel-on-use, and spread and have storage stability.

### [Example 17]

### Moisturizing cream

### <Preparation of cosmetic composition>

A moisturizing cream was obtained by
A: uniformly mixing components 1 to 6,
B: uniformly mixing components 7 to 12,
C: adding B to A and emulsifying.

| | Formulation | (%) |
|---|---|---|
| 1. KSG-340 ^{∗}1 | | 3.0 |
| 2. KF-6048 ^{∗}2 | | 0.2 |
| 3. KSG-44 ^{∗}3 | | 1.0 |
| 4. squalane | | 10.0 |
| 5. jojoba seed oil | | 1.0 |
| 6. olive seed oil | | 0.5 |
| 7. pentylene glycol | | 3.0 |
| 8. BG | | 8.0 |
| 9. dipotassium glycyrrhizinate | | 0.2 |
| 10. sodium citrate | | 0.2 |
| 11. sodium chloride | | 0.5 |
| 12. purified water | | balance |
| | Total | 100.0 |

| | | |
|---|---|---|
| ^{∗}1 mixture of 15% (PEG-15/lauryl dimethicone) crosspolymer (labeling name, INCI name: PEG-15/Lauryl Dimethicone Crosspolymer), 15% (PEG-10/lauryl dimethicone) crosspolymer (labeling name, INCI name: PEG-10/Lauryl Dimethicone Crosspolymer, and 70% squalane (labeling name, INCI name: Squalane) ^{∗}2 cetyl PEG/PPG-10/1 dimethicone (labeling name, INCI name: Cetyl PEG/PPG-10/1 Dimethicone) ^{∗}3 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) | | |

The resulting moisturizing cream was confirmed to show satisfactory water-breaking sensation, feel-on-use, and spread and have storage stability.

### [Example 18]

### Stick foundation

### <Preparation of cosmetic composition>

A stick foundation was obtained by
A: dispersing components 9 to 13 on a three-roll mill,
B: heating components 1 to 8 at 95°C and uniformly mixing them,
C: uniformly mixing components 14 to 17 and A, heating at 85°C,
D: adding C to B, emulsifying, filling a stick container with the emulsion, and slowly cooling.

| | | Formulation | (%) |
|---|---|---|---|
| 1. | KSG-310 ^{∗}1 | | 4.0 |
| 2. | KF-6048 ^{∗}2 | | 0.5 |
| 3. | inulin stearate ^{∗}3 | | 2.5 |
| 4. | ceresin | | 5.5 |
| 5. | KF-56A ^{∗}4 | | 3.0 |
| 6. | polymethylsilsesquioxane ^{∗}5 | | 1.5 |
| 7. | isotridecyl isononanoate | | 2.0 |
| 8. | squalane | | 5.0 |
| 9. | KTP-09W ^{∗}6 | | 6.5 |
| 10. | KTP-09R ^{∗}7 | | 0.3 |
| 11. | KTP-09Y ^{∗}7 | | 0.6 |
| 12. | KTP-09B ^{∗}7 | | 0.1 |
| 13. | DPG | | 6.0 |
| 14. | methylparaben | | 0.1 |
| 15. | sodium citrate | | 0.2 |
| 16. | sodium chloride | | 0.5 |
| 17. | water | | balance |
| | | Total | 100.0 |

| | | | |
|---|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}2 cetyl PEG/PPG-10/1 dimethicone (labeling name, INCI name: Cetyl PEG/PPG-10/1 Dimethicone) ^{∗}3 inulin stearate (labeling name, INCI name: Stearoyl Inulin) ^{∗}4 diphenylsiloxyphenyl trimethicone (labeling name, INCI name: Diphenylsiloxyphenyltrimethicone) ^{∗}5 polymethylsilsesquioxane (labeling name, INCI name: Polymethylsilsesquioxane) ^{∗}6 titanium oxide (labeling name, INCI name: Titanium Dioxide) treated with triethoxysilylethyl polydimethylsiloxyethyl hexyl dimethicone (labeling name, INCI name: Triethoxysilylethyl Polydimethylsiloxyethyl Hexyl Dimethicone) ^{∗}7 iron oxide (labeling name, INCI name: Iron Oxides) treated with triethoxysilylethyl polydimethylsiloxyethyl hexyl dimethicone (labeling name, INCI name: Triethoxysilylethyl Polydimethylsiloxyethyl Hexyl Dimethicone) | | | |

The resulting stick foundation was confirmed to show satisfactory water-breaking sensation, feel-on-use, and spread and have storage stability.

### [Example 19]

### BB cream

### <Preparation of cosmetic composition>

A BB cream was obtained by
A: dispersing components 8 to 16 on a three-roll mill,
B: uniformly mixing components 1 to 7 and A,
C: uniformly mixing components 17 to 21,
D: adding C to B and emulsifying.

| | | Formulation | (%) |
|---|---|---|---|
| 1. | KSG-320 ^{∗}1 | | 4.0 |
| 2. | KF-6048 ^{∗}2 | | 0.5 |
| 3. | KSG-18A ^{∗}3 | | 1.0 |
| 4. | KF-7312J ^{∗}4 | | 0.5 |
| 5. | ethylhexyl methoxycinnamate | | 5.0 |
| 6. | hexyl diethylaminohydroxybenzoylbenzoate | | 1.0 |
| 7. | octocrylene | | 2.0 |
| 8. | KF-56A ^{∗}5 | | 3.0 |
| 9. | cetyl ethylhexanoate | | 2.0 |
| 10. | KP-578 ^{∗}6 | | 0.3 |
| 11. | KTP-09W ^{∗}7 | | 5.0 |
| 12. | KTP-09R ^{∗}8 | | 0.3 |
| 13. | KTP-09Y ^{∗}8 | | 0.6 |
| 14. | KTP-09B ^{∗}8 | | 0.1 |
| 15. | metal soap-treated microparticulate titanium oxide | | 2.0 |
| 16. | silicone-treated microparticulate zinc oxide | | 4.0 |
| 17. | BG | | 8.0 |
| 18. | methylparaben | | 0.1 |
| 19. | sodium citrate | | 0.2 |
| 20. | sodium chloride | | 0.5 |
| 21. | water | | balance |
| | | Total | 100.0 |

| | | | |
|---|---|---|---|
| ^{∗}1 mixture of 25% (PEG-15/lauryl dimethicone) crosspolymer (labeling name, INCI name: PEG-15/Lauryl Dimethicone Crosspolymer) and 75% isododecane (labeling name, INCI name: Isododecane) ^{∗}2 cetyl PEG/PPG-10/1 dimethicone (labeling name, INCI name: Cetyl PEG/PPG-10/1 Dimethicone) ^{∗}3 mixture of 15% (dimethicone/phenyl vinyl dimethicone) crosspolymer (labeling name, INCI name: Dimethicone/Phenylvinyldimethicone Crosspolymer) and 85% diphenylsiloxyphenyl trimethicone (labeling name, INCI name: Diphenylsiloxyphenyltrimethicone) ^{∗}4 mixture of 50% trimethylsiloxysilicic acid (labeling name, INCI name: Trimethylsiloxysilicate) and 50% cyclopentasiloxane (labeling name, INCI name: cyclopentasiloxane) ^{∗}5 diphenylsiloxyphenyl trimethicone (labeling name, INCI name: Diphenylsiloxyphenyltrimethicone) ^{∗}6 (acrylate/ethylhexyl acrylate/dimethicone methacrylate) copolymer (labeling name, INCI name: Acrylates/Ethylhexyl Acrylate/Dimethicone Methacrylate Copolymer) ^{∗}7 titanium oxide (labeling name, INCI name: Titanium Dioxide) treated with triethoxysilylethyl polydimethylsiloxyethyl hexyl dimethicone (labeling name, INCI name: Triethoxysilylethyl Polydimethylsiloxyethyl Hexyl Dimethicone) ^{∗}8 iron oxide (labeling name, INCI name: Iron Oxides) treated with triethoxysilylethyl polydimethylsiloxyethyl hexyl dimethicone (labeling name, INCI name: Triethoxysilylethyl Polydimethylsiloxyethyl Hexyl Dimethicone) | | | |

The resulting BB cream was confirmed to show satisfactory water-breaking sensation, feel-on-use, and spread and have storage stability.

### [Example 20]

### BB cream

### <Preparation of cosmetic composition>

A BB cream was obtained by
A: dispersing components 7 to 11 on a three-roll mill,
B: uniformly mixing components 1 to 6 and A,
C: uniformly mixing components 14 to 21,
D: adding C to B, emulsifying, and adding components 12 and 13 thereto.

| | | Formulation | (%) |
|---|---|---|---|
| 1. | KSG-340 ^{∗}1 | | 3.0 |
| 2. | KF-6048 ^{∗}2 | | 0.5 |
| 3. | KF-6017 ^{∗}3 | | 0.3 |
| 4. | KSG-44 ^{∗}4 | | 1.0 |
| 5. | isodecyl isononanoate | | 10.0 |
| 6. | disteardimonium hectorite | | 0.5 |
| 7. | cetyl ethylhexanoate | | 3.0 |
| 8. | KTP-09W ^{∗}5 | | 5.0 |
| 9. | KTP-09R ^{∗}6 | | 0.3 |
| 10. | KTP-09Y ^{∗}6 | | 0.6 |
| 11. | KTP-09B ^{∗}6 | | 0.1 |
| 12. | SPD-T7 ^{∗}7 | | 2.0 |
| 13. | SPD-Z5 ^{∗}8 | | 4.0 |
| 14. | BG | | 5.0 |
| 15. | hydroxyethyl cellulose | | 0.3 |
| 16. | arbutin | | 0.5 |
| 17. | betaine | | 0.5 |
| 18. | methylparaben | | 0.1 |
| 19. | sodium citrate | | 0.2 |
| 20. | sodium chloride | | 0.5 |
| 21. | water | | balance |
| | | Total | 100.0 |

| | | | |
|---|---|---|---|
| ^{∗}1 mixture of 15% (PEG-15/lauryl dimethicone) crosspolymer (labeling name, INCI name: PEG-15/Lauryl Dimethicone Crosspolymer), 15% (PEG-10/lauryl dimethicone) crosspolymer (labeling name, INCI name: PEG-10/Lauryl Dimethicone Crosspolymer, and 70% squalane (labeling name, INCI name: Squalane) ^{∗}2 cetyl PEG/PPG-10/1 dimethicone (labeling name, INCI name: Cetyl PEG/PPG-10/1 Dimethicone) ^{∗}3 PEG-10 dimethicone (labeling name, INCI name: PEG-10 Dimethicone) ^{∗}4 mixture of 30% (vinyl dimethicone/lauryl dimethicone) crosspolymer (labeling name, INCI name: Vinyl Dimethicone/Lauryl Dimethicone Crosspolymer) and 70% squalane (labeling name, INCI name: Squalane) ^{∗}5 titanium oxide (labeling name, INCI name: Titanium Dioxide) treated with triethoxysilylethyl polydimethylsiloxyethyl hexyl dimethicone (labeling name, INCI name: Triethoxysilylethyl Polydimethylsiloxyethyl Hexyl Dimethicone) ^{∗}6 iron oxide (labeling name, INCI name: Iron Oxides) treated with triethoxysilylethyl polydimethylsiloxyethyl hexyl dimethicone (labeling name, INCI name: Triethoxysilylethyl Polydimethylsiloxyethyl Hexyl Dimethicone) ^{∗}7 dispersion of 45% titanium oxide (labeling name, INCI name: Titanium Dioxide) ^{∗}8 dispersion of 60% zinc oxide (labeling name, INCI name: Zinc Oxide) | | | |

The resulting BB cream was confirmed to show satisfactory water-breaking sensation, feel-on-use, and spread and have storage stability.

### [Example 21]

### Sunscreen cream

### <Preparation of cosmetic composition>

A sunscreen cream was obtained by
A: uniformly mixing components 1 to 8,
B: uniformly mixing components 10 to 15,
C: adding B to A, emulsifying, and adding component 9 thereto.

| | | Formulation | (%) |
|---|---|---|---|
| 1. | KSG-310 ^{∗}1 | | 3.5 |
| 2. | KF-6048 ^{∗}2 | | 0.2 |
| 3 | KF-6038 ^{∗}3 | | 0.1 |
| 4. | KSG-41A ^{∗}4 | | 1.0 |
| 5. | isotridecyl isononanoate | | 2.0 |
| 6. | KF-56A ^{∗}5 | | 5.0 |
| 7. | ethylhexyl methoxycinnamate | | 5.0 |
| 8. | hexyl diethylaminohydroxybenzoylbenzoate | | 2.0 |
| 9. | SPD-Z5 ^{∗}6 | | 15.0 |
| 10. | BG | | 8.0 |
| 11. | ethanol | | 5.0 |
| 12. | phenoxyethanol | | 0.3 |
| 13. | sodium citrate | | 0.2 |
| 14. | sodium chloride | | 0.5 |
| 15. | purified water | | balance |
| | | Total | 100.0 |

| | | | |
|---|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}2 cetyl PEG/PPG-10/1 dimethicone (labeling name, INCI name: Cetyl PEG/PPG-10/1 Dimethicone) ^{∗}3 lauryl PEG-9 polydimethylsiloxyethyl dimethicone (labeling name, INCI name: Lauryl PEG-9 Polydimethylsiloxyethyl Dimethicone) ^{∗}4 alkyl-modified, partially crosslinked organopolysiloxane composition: KSG-41A (crosslinked product ~25% and mineral oil ~75%, by Shin-Etsu Chemical Co., Ltd.) ^{∗}5 diphenylsiloxyphenyl trimethicone (labeling name, INCI name: Diphenylsiloxyphenyltrimethicone) ^{∗}6 dispersion of 60% zinc oxide (labeling name, INCI name: Zinc Oxide) | | | |

The resulting sunscreen cream was confirmed to show satisfactory water-breaking sensation, feel-on-use, and spread and have storage stability.

### [Example 22]

### Sunscreen cream

### <Preparation of cosmetic composition>

A sunscreen cream was obtained by
A: uniformly mixing components 1 to 6,
B: uniformly mixing components 9 to 15,
C: adding B to A, emulsifying, and adding components 7 and 8 thereto.

| | | | |
|---|---|---|---|
| | | Formulation | (%) |
| 1. | KSG-820 ^{∗}1 | | 3.5 |
| 2. | KF-6048 ^{∗}2 | | 0.5 |
| 3. | KSG-42A ^{∗}3 | | 3.0 |
| 4. | KF-56A ^{∗}4 | | 3.0 |
| 5. | isododecane | | 12.0 |
| 6. | isotridecyl isononanoate | | 3.0 |
| 7. | KSP-105 ^{∗}5 | | 1.0 |
| 8. | SPD-Z5 ^{∗}6 | | 10.0 |
| 9. | sodium hyaluronate (2% solution) | | 5.0 |
| 10. | BG | | 5.0 |
| 11. | ethanol | | 5.0 |
| 12. | phenoxyethanol | | 0.3 |
| 13. | sodium citrate | | 0.2 |
| 14. | magnesium sulfate | | 0.5 |
| 15. | purified water | | balance |
| | | Total | 100.0 |

| | | | |
|---|---|---|---|
| ^{∗}1 mixture of 25% (lauryl dimethicone/polyglycerin-3) crosspolymer (labeling name, INCI name: Lauryl Dimethicone/Polyglycerin-3 Crosspolymer) and 75% isododecane (labeling name, INCI name: Isododecane) ^{∗}2 cetyl PEG/PPG-10/1 dimethicone (labeling name, INCI name: Cetyl PEG/PPG-10/1 Dimethicone) ^{∗}3 mixture of 80% vinyl dimethicone/lauryl dimethicone crosspolymer (labeling name, INCI name: Vinyl Dimethicone/Lauryl Dimethicone Crosspolymer) and 20% isododecane (labeling name, INCI name: Isododecane) ^{∗}4 diphenylsiloxyphenyl trimethicone (labeling name, INCI name: Diphenylsiloxyphenyltrimethicone) ^{∗}5 vinyl dimethicone/methicone silsesquioxane crosspolymer (labeling name, INCI name: Vinyl Dimethicone/Methicone Silsesquioxane Crosspolymer) ^{∗}6 dispersion of 60% zinc oxide (labeling name, INCI name: Zinc Oxide) | | | |

The resulting sunscreen cream was confirmed to show satisfactory water-breaking sensation, feel-on-use, and spread and have storage stability.

### [Example 23]

### Sunscreen lotion

### <Preparation of cosmetic composition>

A sunscreen lotion was obtained by
A: uniformly mixing components 1 to 11,
B: uniformly mixing components 13 to 18,
C: adding B to A, emulsifying, and adding component 12 thereto.

| | | Formulation | (%) |
|---|---|---|---|
| 1. | KSF-310 ^{∗}1 | | 3.5 |
| 2. | KF-6048 ^{∗}2 | | 0.5 |
| 3. | KSG-18A ^{∗}3 | | 5.0 |
| 4. | disteardimonium hectorite | | 0.5 |
| 5. | C13-15 alkane | | 2.0 |
| 6. | isononyl isononanoate | | 2.0 |
| 7. | ethylhexyl palmitate | | 2.0 |
| 8. | neopentyl glycol diethylhexanoate | | 2.0 |
| 9. | KF-56A ^{∗}4 | | 5.0 |
| 10. | ethylhexyl methoxycinnamate | | 5.0 |
| 11. | hexyl diethylaminohydroxybenzoylbenzoate | | 2.0 |
| 12. | SPD-Z5 ^{∗}5 | | 15.0 |
| 13. | BG | | 8.0 |
| 14. | ethanol | | 5.0 |
| 15. | phenoxyethanol | | 0.3 |
| 16. | sodium citrate | | 0.2 |
| 17. | sodium chloride | | 0.5 |
| 18. | purified water | | balance |
| | | Total | 100.0 |

| | | | |
|---|---|---|---|
| ^{∗}1 mixture of 30% (PEG-15/lauryl dimethicone) crosspolymer (labeling name, INCI name: PEG-15/Lauryl Dimethicone Crosspolymer) and 70% mineral oil (labeling name, INCI name: Mineral Oil) ^{∗}2 cetyl PEG/PPG-10/1 dimethicone (labeling name, INCI name: Cetyl PEG/PPG-10/1 Dimethicone) ^{∗}3 mixture of 15% (dimethicone/phenylvinyl dimethicone) crosspolymer (labeling name, INCI name: Dimethicone/Phenylvinyldimethicone Crosspolymer) and 85% diphenylsiloxyphenyl trimethicone (labeling name, INCI name: Diphenylsiloxyphenyltrimethicone) ^{∗}4 diphenylsiloxyphenyl trimethicone (labeling name, INCI name: Diphenylsiloxyphenyltrimethicone) ^{∗}5 dispersion of 60% zinc oxide (labeling name, INCI name: Zinc Oxide) | | | |

The resulting sunscreen lotion was confirmed to show satisfactory water-breaking sensation, feel-on-use, and spread and have storage stability.

## Claims

1. A water-in-oil cosmetic composition comprising:
(a) 0.1 to 3% by weight of at least one silicone selected from among alkyl-modified, partially crosslinked polyether-modified silicones and alkyl-modified, partially crosslinked polyglycerin-modified silicones,
(b) 0.05 to 0.8% by weight of a non-crosslinked alkyl-branched polyether-modified silicone,
(c) 30 to 90% by weight of an aqueous component, and
(d) 5 to 30% by weight of an oil component exclusive of silicone oil.

2. The water-in-oil cosmetic composition of claim 1, further comprising (e) an alkyl-modified, partially crosslinked organopolysiloxane exclusive of component (a).

3. The water-in-oil cosmetic composition of claim 1 or 2 wherein component (d) contains a hydrocarbon oil and a low polar ester oil in a total amount of at least 80% by weight.

4. The water-in-oil cosmetic composition of any one of claims 1 to 3 wherein component (a) is a swollen product which is previously swollen in a hydrocarbon oil.

5. The water-in-oil cosmetic composition of any one of claims 1 to 4 wherein component (b) is cetyl PEG/PPG-10/1 dimethicone as defined by the INCI name.
